# EUROPEAN PATENT SPECIFICATION

(11) **EP 3 081 263 B1**
(45) Date of publication and mention of the grant of the patent: **12.02.2020**
(21) Application number: 15163949.9
(22) Date of filing: 17.04.2015
(51) Int. Cl.: A61K 35/30, A61P 27/06, A61K 9/00, A61M 5/172, A61M 27/00, A61M 5/142, A61K 38/19, A61P 25/28

(54) **COMPOSITIONS AND TOOLS FOR TREATING GLAUCOMA**
ZUSAMMENSETZUNGEN UND WERKZEUGE ZUR BEHANDLUNG VON GLAUKOM
COMPOSITIONS ET OUTILS POUR LE TRAITEMENT DU GLAUCOME

(43) Date of publication of application: 19.10.2016
(73) Proprietor: P&X Medical NV, 8020 Oostkamp (BE)
(72) Inventor: Wostyn, Peter, 8670 Oostduinkerke (BE)
(74) Representative: Nederlandsch Octrooibureau

(56) References cited:
- US-A1- 2005 090 549
- US-A1- 2013 238 015

## Description

### FIELD OF THE INVENTION

The present application relates generally to the field of glaucoma and methods and medical devices for the treatment thereof. More particularly, the application provides methods and tools for treating and/or preventing glaucoma by increasing intracranial pressure and/or CSF turnover. The application further provides implantable, pump-assisted devices, capable of increasing intracranial pressure and/or CSF turnover.

### BACKGROUND

Glaucoma is one of the leading causes of irreversible blindness. The most common type of glaucoma is primary open-angle glaucoma (POAG), which is a progressive optic neuropathy with characteristic structural changes in the optic nerve head and corresponding visual field defects. In the glaucomatous optic nerve, cupping of the optic disc reflects a loss of retinal ganglion cell (RGC) axons and a posterior bowing of the lamina cribrosa (forming the anatomic floor of the optic nerve head), accompanied by extensive remodeling of the optic nerve head.

The brain and spinal cord are encased within the cranium and vertebral column inside a thin membrane known as the arachnoid. The volume of the intracranial space is on average about 1700 ml including volumes of approximately 1400 ml of brain, approximately 150 ml of intracranial blood; and approximately 150 ml of CSF. CSF circulates within the subarachnoid space and is formed principally by the choroid plexuses, which secrete about 80% of the total volume. The sources of the remainder of CSF are the vasculature of the subependymal regions, and the pia mater. The total volume of CSF is renewed several times per day, so that about 500 ml are produced every 24 hours. CSF is absorbed through the arachnoid villi, located principally over the superior surfaces of the cerebral hemispheres. Some villi also exist at the base of the brain and along the roots of the spinal nerves. The absorptive processes include bulk transport of large molecules and as well as diffusion across porous membranes of small molecules.

Raised intraocular pressure is recognized as one of the most important risk factors for POAG and lowering it remains the only current therapeutic approach for slowing optic nerve damage and visual field progression in glaucoma patients. Known glaucoma therapies include medicines (e.g., prostaglandin analogues, beta-blockers, carbonic anhydrase inhibitors, and alpha-agonists), laser surgery (e.g., laser trabeculoplasty), and incisional surgery (e.g., trabeculectomy, deep sclerectomy, viscocanalostomy, and glaucoma drainage implants).

Therapy typically starts from the least invasive options, which usually involve the administration of medication. However, the administration of medication often fails for various reasons. Indeed, medicaments for the treatment of POAG typically lower the IOP by at most about 25% to 30%, which can be insufficient. Some glaucoma patients show disease progression despite of the administration of medicaments. Moreover, topical medications for glaucoma can cause side effects such as precipitation of asthma, bradycardia, impotence, and decreased exercise tolerance. There is also a significant problem in compliance with glaucoma medications due to frequent dosing regimens. Incisional surgery is usually required when (topical) glaucoma medication and/or laser surgery fail. However, current incisional surgery techniques for treating glaucoma can lead to various complications including but not limited to choroidal effusion, hypotony maculopathy, suprachoroidal haemorrhage, and bleb infections.

Accordingly, there is a need for an alternative glaucoma treatment which mitigates at least one of the above problems.

A number of implantable pumps have been described in the art. U.S. Pat. Pub. No. 2005/0090549 (Hildebrand et al.) describes a system and method that may be used to treat pain by administering gabapentin to cerebrospinal fluid (CSF) of a patient. The system includes a pump, a catheter and a reservoir containing an effective amount of gabapentin to treat pain in the patient by pumping the gabapentin through the catheter into CSF. U.S. Pat. Pub. No. 201110021469 (Meythaler et al.) describes intrathecal delivery of baclofen to reduce spasticity. Meythaler describes using refillable programmable pump systems that are implantable and provide continuous infusion of baclofen directly into CSF of a patient. US 2013/238015 discloses a device useful for the treatment of glaucoma. The device monitors intraocular pressure or cerebrospinal fluid pressure and treats glaucoma by lowering intraocular pressure or increasing cerebrospinal fluid pressure.

However, neither Hildebrand nor Meythaler disclose or suggest a method or system which involves increasing intracranial pressure, nor suggest the use of such a system to treat glaucoma.

### SUMMARY OF THE INVENTION

The invention is defined by the claims. Any subject-matter falling outside the scope of the claims is provided for information purposes only. Furthermore, any references in the description to methods of treatment refer to the compounds, pharmaceutical compositions and medicaments of the present invention for use in a method of treatment of the human or animal body by therapy. The invention is directed to a composition comprising CSF or a CSF-like composition for use in the prevention and/or treatment of glaucoma, wherein the CSF-like composition is artificial CSF (aCSF), wherein aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM.

Described herein are methods and tools for the prevention and treatment of glaucoma. In particular, the methods comprise increasing intracranial pressure and/or CSF turnover by the administration of artificial CSF or CSF-like solutions.

Further described herein is an apparatus for infusing artificial cerebrospinal fluid (CSF) to the CSF of a patient, in order to increase the intracranial pressure and/or the cerebrospinal fluid turnover in said patient. The principle of the methods and tools described herein is based on the observation that glaucoma may not only be associated with raised IOP, but also with a reduced intracranial pressure (ICP). The present inventor proposes the infusion of artificial CSF for the treatment and/or prevention of glaucoma. In particular embodiments this can be achieved through an implantable pump, whereby artificial CSF is infused into the intrathecal space or into the cerebral ventricles. The outlet end of the inflow catheter may be disposed in any region of the spine, including the cervical region, the thoracic region, the lumbar region etc.. It is envisaged that this provides a protective effect for the optic nerve by increasing the ICP and/or the CSF turnover and clearance.

Provided herein is a method of prevention and/or treatment of glaucoma in a patient which comprises administering artificial CSF directly or indirectly into the cerebral ventricles or the intrathecal space of said patient. More particularly the method comprises administering artificial CSF so as to ensure an increase in ICP and/or to increase CSF turnover. In particular embodiments, the method comprises reducing the trans-lamina cribrosa pressure difference (TLCPD; i.e. IOP minus ICP), preferably to a value of about 4 mm Hg, or less such as to a value of 2 or 1 mm Hg. In particular embodiments, the method comprises ensuring a ICP of between 11 and 16 mm Hg, more particularly an ICP of about 15 mm Hg, when measured in the lateral decubitus position. Additionally or alternatively, the methods provided herein comprise ensuring an increase in the total CSF turnover in the patient. The optimal infusion rate of CSF will be dependent on the natural daily absorption of CSF by the patient to allow the body to readily absorb CSF and maintain an adequate ICP. In particular embodiments, the pump may maintain an infusion rate of the fluid in the range of 0.05-0.1 ml/min, 0.1-0.2 ml/min, 0.2-0.42 ml/min, 0.42-0.7ml/min or even up to 0.7-1.04 ml/min (1.5 L/day). In particular embodiments a turnover is ensured of about 4.0 volumes/day. In particular embodiments, the methods comprise administering the artificial CSF to the intrathecal or subarachnoid space or the cerebral ventricles of said patient by supplementing said patient's CSF with artificial CSF. More particularly this is ensured by way of an apparatus capable of infusing fluid with an implantable pump, more particularly artificial CSF directly or indirectly into the intrathecal space and/or the cerebral ventricles of said patient.

Also provided herein is an apparatus for infusing fluid into a body cavity, more particularly the intrathecal or subarachnoid space or the cerebral ventricles, the apparatus comprising:
- an implantable pump;
- a reservoir containing artificial cerebrospinal fluid;
- an infusion catheter having an inlet end coupled to the reservoir, and an outlet end coupled to the implantable pump; and
- an inflow catheter having an outlet end configured to be disposed in fluid communication with a body cavity, and an inlet end coupled to the implantable pump;
wherein the implantable pump is configured to selectively move artificial cerebrospinal fluid from the reservoir through the infusion catheter and the inflow catheter to the intrathecal space or the cerebral ventricles at a rate and volume sufficient to increase the intracranial pressure and/or the cerebrospinal fluid turnover in a patient.

In particular embodiments, the apparatus further comprises a microcontroller that controls operation of the implantable pump. In particular embodiments, the apparatus comprises a pressure sensor disposed in communication with the inflow catheter to monitor pressure of cerebrospinal fluid, wherein the microcontroller is configured to activate the implantable pump responsive to an output of the pressure sensor.

In particular embodiments the microcontroller is configured to activate the implantable pump so as to ensure a CSF pressure of between 11 and 16 mm Hg, more particularly an ICP of about 15 mm Hg, when measured in the lateral decubitus position. In particular embodiments, the apparatus comprises a feedback mechanism based on said pressure sensor, which ensures that the CSF pressure does not exceed 15 mm Hg. In particular embodiments the microcontroller of the apparatus ensures an increased CSF turnover. In particular embodiments, the CSF turnover is increased to about 4.0 volumes/day.

In particular embodiments of the apparatus envisaged herein, the reservoir contains artificial cerebrospinal fluid.

The above and other characteristics, features and advantages of the present invention will become apparent from the following detailed description, which illustrates, by way of example, the principles of the invention.

### BRIEF DESCRIPTION OF THE DRAWINGS

The following description of the figures of specific embodiments of the invention is merely exemplary in nature and is not intended to limit the present teachings, their application or uses. Throughout the drawings, corresponding reference numerals indicate like or corresponding parts and features.
FIG. 1A is a schematic view of the implantable components connected to a brain and an external reservoir according to an embodiment of the invention. FIG. 1B is a schematic view of the implantable components connected to a spinal cord and an external reservoir according to an embodiment of the invention. FIG. 1C is a schematic view of the implantable components connected to a brain and an implantable reservoir according to an embodiment of the invention.
FIG. 2 is a schematic diagram of a fluid infusion system according to an embodiment of the present invention.
FIG. 3 is a perspective view of the outlet end of the inflow catheter of the fluid infusion system according to an embodiment of the invention.
FIGS. 4A and 4B are, respectively, a perspective view of the implantable pump for use in the fluid infusion system and a cross-sectional view of an implantable pump mechanism for use within the fluid infusion system according to an embodiment of the invention.
FIGS. 5A, 5B, and 5C illustrate cross-sectional views of alternative one-way valves to control the direction of fluid flow within the fluid infusion system according to an embodiment of the invention.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms "first", "second", "third" and the "like" in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Reference throughout this specification to "one embodiment" or "an embodiment" means that a particular feature, structure or characteristic described in connection with the embodiment is included in at least one embodiment of the present description. Thus, appearances of the phrases "in one embodiment" or "in an embodiment" in various places throughout this specification are not necessarily all referring to the same embodiment. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the features of the claimed embodiments can be used in any combination.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that each value as used herein is itself also specifically, and preferably, disclosed.

The recitation of numerical ranges by endpoints includes all numbers and fractions subsumed within the respective ranges, as well as the recited endpoints.

CSF flows from the brain ventricles into interconnecting chambers, namely, the cisterns and the subarachnoid spaces, including the SAS of the optic nerves. The optic nerve, a white matter tract of the central nervous system, is ensheathed in all three meningeal layers and surrounded by cerebrospinal fluid (CSF) in the subarachnoid space (SAS) with a pressure equivalent to intracranial pressure (ICP). Thus, in addition to intraocular pressure (IOP), the optic nerve is exposed to the ICP. The lamina cribrosa, the perforated region of the sclera through which the nerve fibers of the optic nerve pass as they exit the eye, separates these two pressurized regions. The difference between the posteriorly directed IOP and anteriorly directed ICP across the lamina cribrosa is known as the trans-lamina cribrosa pressure difference (TLCPD).The term "intracranial pressure" or "ICP" as used herein thus refers to the pressure of cerebrospinal fluid (CSF) within the skull and thus in the brain tissue and CSF and is also referred to as "CSF pressure". The CSF pressure as assessed by lumbar puncture correlates with ICP, and thus the terms CSF pressure and ICP are used interchangeably. The ICP is built up by the equilibrium between the production and outflow of CSF. If the intracranial compliance is assumed to be constant, the steady-state ICP can be described by a simplified equation: ICP = I_{f} x Rₒᵤₜ + Pₛₛ, where I_{f} is CSF formation rate, Rₒᵤₜ is outflow resistance, and Pₛₛ is sagittal sinus pressure. ICP is measured in millimetres of mercury (mmHg). At rest it is normally between 5-15 mmg Hg for an adult when measured by lumbar puncture in the lateral decubitus position. Accordingly, the values of ICP (or CSF pressure) referred to herein refer to values when measured in the lateral decubitus position.

The term "intraocular pressure" or "IOP" as used herein refers to the fluid pressure within the eye. It is measured in millimetres of mercury (mmHg). Normally the IOP ranges from 11 to 21 mmHg with a mean of 16 mmHg.

The "trans-lamina cribrosa pressure difference" or "TLCPD" is the difference between the posteriorly directed IOP and the anteriorly directed ICP across the lamina cribrosa.

The pressure drop that occurs across the lamina cribrosa (IOP-ICP) increases with elevation of IOP or reduction of ICP. Indeed, from a mechanical perspective, a similar posteriorly directed force is caused by either a lower pressure on the CSF side of the lamina or a higher pressure on the intraocular side.

A CSF-like solution as used herein refers to a solution that consists essentially of CSF or artificial CSF.

The term "artificial CSF" (aCSF) as used herein refers to a solution that closely matches the electrolyte concentrations of cerebrospinal fluid. Typically, the artificial CSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM. In one example, the artificial CSF comprises sodium ions at a concentration of 150 mM, potassium ions at a concentration of 3 mM, calcium ions at a concentration of 1.4 mM, magnesium ions at a concentration of 0.8 mM, phosphor ions at a concentration of 1 mM, chloride ions a concentration of 155 mM. aCSFs have been described in the art and include, but are not limited to Elliot's solutions A and B and ARTCEREB ™.

Typically where reference is made in the description to the administration of CSF, it is intended to refer to a CSF-like solution or to CSF which is (at least partially) of foreign origin (i.e. not from the patient).

In particular embodiments, the CSF may further comprise one or more therapeutic agents, for example agents for reducing the IOP and/or increasing the ICP. For example specific peptides such as angiotensin have been shown to facilitate the rise in CSF pressure upon CSF infusion.

The term "intrathecal space" also referred to as the subarachnoid space (SAS) is the fluid-filled area located between the innermost layer of covering (the pia mater) of the spinal cord and the middle layer of covering (the arachnoid mater).

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Provided herein are methods and devices for treating glaucoma. More particularly, provided herein are methods and devices for increasing the ICP (and thus reducing TLCPD) and/or augmenting the rate of CSF turnover, for the treatment and/or prevention of glaucoma. More specifically, the methods and tools described herein involve the administration of CSF or a CSF-like solution (such as artificial CSF) directly or indirectly to the cerebral ventricles and/or to the intrathecal space around the spinal cord. By delivering CSF or artificial CSF to the subarachnoid region, the ICP is increased (or the TLCPD is decreased) and/or the rate of CSF turnover and clearance in the subarachnoid space of the optic nerve is increased (thereby enhancing removal of potentially neurotoxic waste products that accumulate in the optic nerve), thus ensuring the treatment of glaucoma.

Indeed, glaucoma can be prevented or treated from the intracranial compartment side of the lamina instead of, or in addition to, lowering IOP. More particularly, the present inventor has found that reduced ICP contributes to glaucoma via a mismatch in pressures across the lamina cribrosa (TLCPD), such that lowering the TLCPD by manipulation of ICP by infusion of CSF can be used to prevent and/or treat glaucoma. Moreover, the presented treatment allows an enhancement of the rate of CSF turnover which is believed to provide an additional or alternative beneficial effect for the prevention and treatment of glaucoma.

Provided herein are compositions for use in methods of prevention and/or treatment of glaucoma in a patient in need thereof and corresponding methods of treatment and prevention. More particularly, a composition comprising CSF or a CSF-like solution such as artificial CSF is provided for use in the prevention or treatment of glaucoma. In particular embodiments, the methods of prevention and/or treatment of glaucoma in a patient comprise administering CSF or a CSF-like solution to the intrathecal space or subarachnoid space or the cerebral ventricles of said patient. More particularly, the artificial CSF is administered to the intrathecal space surrounding the spinal cord. Indeed, the administration of CSF or a CSF-like solution can be done locally, in the vicinity of the subarachnoid space of the optic nerve, but in most embodiments the same effect can be achieved less invasively by infusion more remotely, i.e. intrathecally anywhere along the spinal cord, including the cervical region, the thoracic region, the lumbar region etc.

More particularly the method comprises administering CSF or a CSF-like solution to a patient in need thereof so as to ensure an increase in ICP and/or to increase CSF turnover. In particular embodiments, the method comprises infusing CSF or a CSF-like solution to the intrathecal space or the cerebral ventricles so as to reduce the TLCPD, preferably to less than 4mm Hg, or even lower to 1 or 2 mmHg. In particular embodiments, the method comprises infusion of CSF or a CSF-like solution such as artificial CSF into the intrathecal or subarachnoid space of a patient to ensure an ICP of between 11 and 16 mm Hg, more particularly up to 15 mm Hg. However, preferably the ICP is not raised above the IOP. Additionally or alternatively, the methods provided herein comprise ensuring an increase in the total CSF turnover. The turnover of CSF decreases substantially with aging and thus the degree of increase in turnover will need to take into consideration the age of the patient. In a young adult, it is envisaged that the turnover is ideally about 4.0 volumes/day. In particular embodiments, the methods comprise administering the CSF or CSF-like solution to the intrathecal or subarachnoid space of said patient by supplementing said patient's CSF with CSF or a CSF-like solution such as artificial CSF. More particularly this is ensured by way of an implantable apparatus configured for infusing fluid, more particularly CSF or a CSF-like solution into the intrathecal or subarachnoid space of said patient.

In particular embodiments, it is envisaged that the CSF or CSF-like solution may comprise a drug associated with intracranial hypertension.

Accordingly, also provided herein is an apparatus such as an infusion pump for infusing CSF or a CSF-like solution into a body cavity, more particularly into the intrathecal or subarachnoid space. The apparatus described herein can be used for the treatment and/or prevention of glaucoma, more particularly open-angle glaucoma. Intrathecal infusion pumps are currently widely used for management of chronic pain (morphine pump) and spasticity (baclofen pump). In particular embodiments, the apparatus for infusing fluid into the intrathecal or subarachnoid space of a patient the apparatus comprises an implantable pump, a reservoir for containing artificial cerebrospinal fluid, an infusion catheter having an inlet end coupled to the reservoir, and an outlet end coupled to the implantable pump and an inflow catheter. In particular embodiments the inflow catheter has an outlet end configured to be disposed in fluid communication with said intrathecal or subarachnoid space, and an inlet end coupled to the implantable pump; typically the implantable pump is configured to selectively move artificial cerebrospinal fluid from the reservoir through the infusion catheter and the inflow catheter to the intrathecal or subarachnoid space at a rate and volume sufficient to increase the intracranial pressure and/or the cerebrospinal fluid turnover in a patient. More particularly the rate and volume of CSF infusion are adjusted so as to reduce TLCPD to a value of about 4mm Hg or less, or even to 2 mm Hg or 1 mm Hg (dependent on the patient). In particular embodiments, the rate and volume of artificial CSF infusion ensures an ICP of between 11 and 16 mm Hg, more particularly up to 15 mm Hg.

The infusion rate ensured by the pump will be determined based on different factors, including the CSF absorption rate of the patient. In particular embodiments, the infusion rate is adjusted to ensure an increased turnover of CSF in the patient. In particular embodiments the pump is configured to ensure a CSF infusion rate in the range of 0.05-0.1 ml/min, 0.1-0.2 ml/min, 0.2-0.42 ml/min, 0.42-0.7ml/min or even up to 0.7-1.04 ml/min (1.5 L/day). In particular embodiments the infusion rate ensures a turnover of about 4.0 volumes/day.

In particular embodiments, the apparatus comprises a microcontroller that controls operation of the implantable pump. In particular embodiments, the microcontroller regulates the flow of the CSF or CSF-like solution through the inflow catheter.

In further embodiments, the apparatus comprises a flow sensor disposed in communication with the inflow catheter to monitor the volume and flow rate of artificial CSF pumped into the intrathecal space or cerebral ventricles, wherein the microcontroller is configured to activate the implantable pump responsive to an output of the pressure sensor.

The application further provides a combination of the apparatus as described herein and an implantable pressure sensor to monitor intracranial or CSF pressure in the patient. In particular embodiments, the sensor is not physically linked to the rest of the apparatus but can be implanted intrathecally or in the cerebral ventricles. In particular embodiments, the microcontroller of the apparatus is configured to activate or deactivate the implantable pump responsive to an output of the pressure sensor. In particular embodiments, the microcontroller is configured to activate and deactivate the implantable pump so as to ensure a constant intracranial pressure of between 11 and 16 mm Hg, more particularly up to 15 mm Hg. In particular embodiments, the apparatus comprises a feedback mechanism based on the output of the pressure sensor, which ensures that the intracranial pressure does not exceed 15 mm Hg.

Further described herein is the treatment and/or prevention of glaucoma by lowering the TLCPD by increasing the ICP and/or by facilitating CSF turnover which involves the infusion of CSF or a CSF-like solution as described herein. In particular embodiments, this is ensured by the use of an apparatus as described herein. Although the implantation of a CSF pump is a relatively invasive intervention, it provides a worthwhile alternative for or addition to existing therapies, especially for patients for whom non-invasive treatment options are ineffective. In particular embodiments, the TLCPD may be lowered by increasing the ICP. More particularly, described herein is a method for treating and/or preventing glaucoma, more particularly open-angle glaucoma in a patient, comprising administering CSF or a CSF-like solution intrathecally or into the cerebral ventricles of said patient. In particular embodiments, said method comprises providing an apparatus as described herein comprising an implantable pump, a reservoir for containing artificial cerebrospinal fluid, an infusion catheter having an inlet end coupled to the reservoir, and an outlet end coupled to the implantable pump; and an inflow catheter having an outlet end configured to be disposed in fluid communication with said intrathecal or subarachnoid space or the cerebral ventricles, and an inlet and further comprising the steps of:
- providing cerebrospinal fluid or a CSF-like solution in said reservoir;
- coupling the inflow catheter to a region of a body cavity, more particularly an intrathecal space or the cerebral vesicles; and
- activating the implantable pump to pump the cerebrospinal fluid or CSF-like fluid from the reservoir through the infusion catheter and the inflow catheter to the body cavity at a rate and volume sufficient to increase the ICP and/or to increase the CSF turnover.

In particular embodiments, the body cavity is the subarachnoid space, one of the lateral ventricles, or the central canal of the spinal cord.

In particular embodiments, the pump is surgically placed under the skin of the abdomen, and delivers small, CSF or CSF-like fluid through a catheter directly into the CSF locally present. The present inventor has found that pumps may be provided for infusing artificial CSF, in order to increase ICP with the aim of treating glaucoma.

The methods and tools described herein are particularly suitable for the prevention and/or treatment of glaucoma. Prevention of glaucoma can be envisaged in patients susceptible to glaucoma such as patients having reduced intracranial pressure and/or increased TLCPD. Examples of risk factors associated with glaucoma include but are not limited to elevated IOP, low ICP, age, gender, high myopica etc. Long term use of topical and systemic steroids produces secondary open-angle glaucoma by causing an increase in IOP.

In particular embodiments, the application envisages determining one or more of the IOP, TLCPD and/or ICP in a patient prior to the administration according to the methods described herein. This step can be ensured in order to determine the suitability of the methods of the invention for the prevention and/or treatment of glaucoma. Additionally or alternatively it can be used to determine the optimal infusion rate of CSF.

Methods applied for non-invasive estimation of ICP are known in the art and include transcranial Doppler ultrasonography, tympanic membrane displacement, ophthalmodynamometry, measurement of the orbital CSF space around the optic nerve, two-depth transcranial Doppler technology and others. Two-Depth Transorbital Doppler (TDTD) measurement of intracranial pressure quantitative absolute (ICP) value relies on the same fundamental principle as used to measure blood pressure with a sphygmomanometer. The TDTD method uses Doppler ultrasound to translate pressure balance principle of blood pressure measurement with a sphygmomanometer to the measurement of ICP. The ophthalmic artery (OA), which is a vessel with intracranial and extracranial segments, is used as pressure sensor and as a natural pair of scales for absolute ICP value in mmHg or mmH₂O measurement. Blood flow in the intracranial OA segment is affected by intracranial pressure, while flow in the extracranial (intraotbital) OA segment is influenced by the externally applied pressure (Pe) to the eyeball and orbital tissues.

### Exemplary infusion system according to the invention

In FIG. IA, infusion system 100 comprises infusion catheter 30 connecting reservoir 60 to pump 50, and inflow catheter 40 connecting pump 50 to cerebral ventricle V of brain B of the patient. While FIG. IA depicts inflow catheter 40 connecting pump 50 to the patient's brain, one skilled in the art would understand that inflow catheter may be similarly connected to another source of CSF including the patient's spine. System 100 provides a unidirectional path for movement of artificial CSF to flow from reservoir 60 to brain B. Referring to FIG. IA, artificial CSF from reservoir 60 is drawn into inlet end 32 of infusion catheter 30 by pump 50, and expelled through outlet end 44 of outlet catheter 40 into brain B. Alternatively, outlet end 44" may be disposed in the spinal cord as depicted in FIG. IB. While the outlet end of the inflow catheter is illustratively disposed in the cervical region of the spine, the outlet end of the inflow catheter may be disposed in any region of the spine, including the lumbar region, the thoracic region, etc. One-way valve 70 is positioned along infusion catheter 30 or inflow catheter 40 to prevent back flow of fluid through system 100. Optional bacterial filter 85 may be positioned along infusion catheter 30, inflow catheter 40, or disposed within the housing of pump 50 to destroy harmful bacteria and prevent bacteria from migrating through system 100 to brain B. Alternatively, or in addition, the components of system 100 may be coated or impregnated with an antibacterial or antimicrobial coating to reduce the risk of infection.

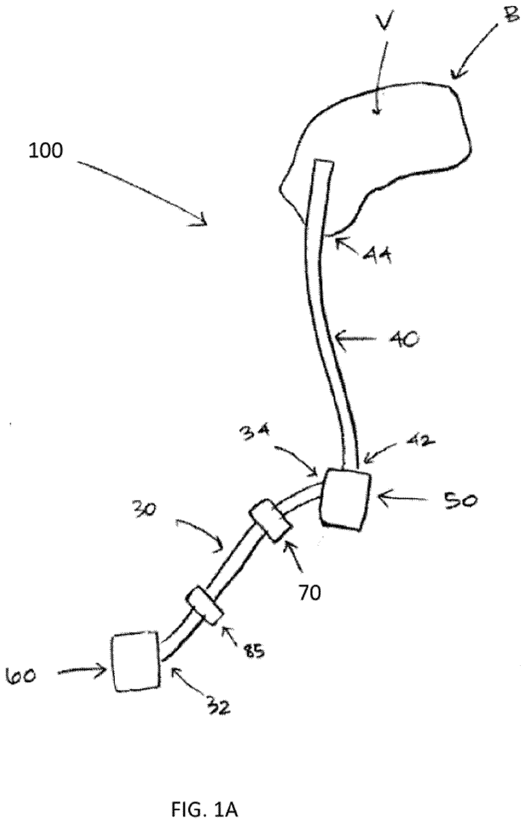

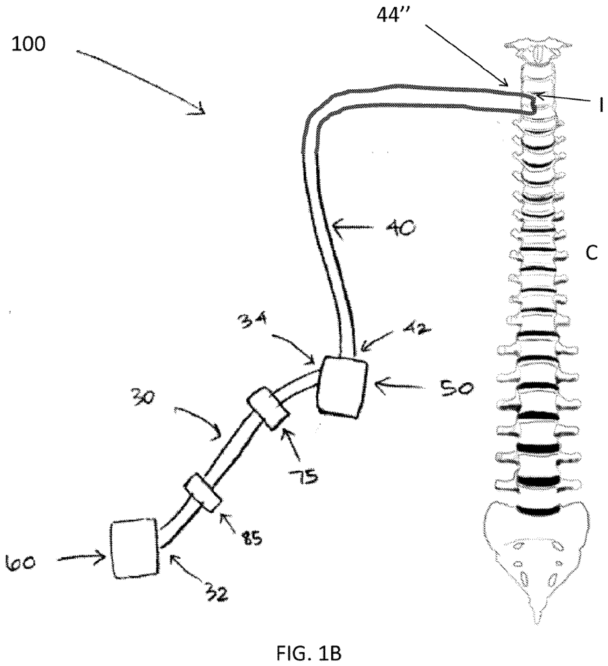

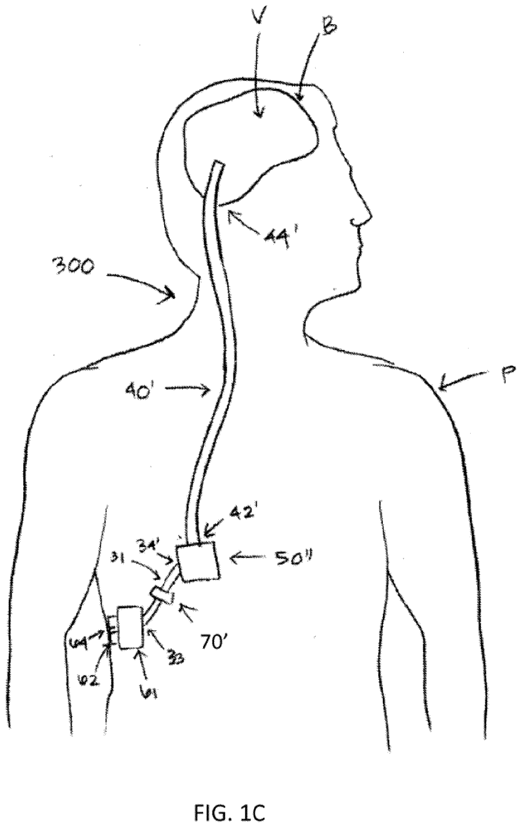

In particular embodiments, infusion catheter 30, inflow catheter 40, and pump 50 may be implanted separately and then coupled together during implantation of pump 50. For example, catheters 30 and 40 may be separately implanted using a tunneling technique to place outlet end 44 of inflow catheter 40 in communication with a different region of the source of CSF. Outlet end 34 of infusion catheter 30, and inlet end 42 of inflow catheter 40 then may be lead to the site for implantation of pump 50, and coupled to the pump prior to implantation. Reservoir 60 may be secured to a holder such as a belt and worn by the patient allowing the patient to transport the reservoir with mobility or reservoir 60 may be connected to infusion catheter 30 like an IV bag. Alternatively, reservoir 61 may be implantable under the skin of patient P, as depicted in FIG. 1C. In an alternative embodiment, one or more of infusion catheter 30, inflow catheter 40, and pump 50 may be coupled together prior to implantation and implanted together.

Referring to FIG. 1C, reservoir 61 also may be implanted separately from infusion catheter 31, inflow catheter 40' and pump 50". For example, inlet end 33 of infusion catheter 31 is placed in communication with reservoir 61 prior to being coupled to pump 50". Reservoir 61 may be configured in any form suitable for placement under the skin so that it is capable of receiving artificial CSF. For example, reservoir 61 may comprise septum 62 fluidly connected to reservoir 61 and/or port opening 64 to receive artificial CSF via a syringe. Conveniently, the form of reservoir 61 may be similar or identical to conventional implantable reservoirs of the type used for delivering a liquid therapeutic substance to a delivery site, such as that described in U.S. Pat. No. 8,348,909 to Haase. Suitable reservoirs that may be incorporated into systems constructed according to the present invention are available from commercial suppliers, such as Medtronic PS Medical, Goleta, California.

As will be understood, catheters 30 and 40 comprise biocompatible materials, and may be provided in standard lengths or a single length that may be cut to size to fit a particular patient's anatomy during the implantation procedure. Each connection in system 100 preferably includes a fluid-tight seal and may be accomplished through any variety of methods as known to one of skill in the art.

Infusion catheter 30 and inflow catheter 40 may be formed from a resilient material, such as implant grade silicone or reinforced silicone tubing. The catheters may be reinforced along a portion of their length or along the entire length of the catheters. Reinforcement of the tubing may be accomplished via ribbon or wire braiding or lengths of wire or ribbon embedded or integrated within or along the tubing. The braiding or wire may be fabricated from metals such as stainless steels, superelastic metals such as nitinol, or from a variety of suitable polymers.

Outlet end 44 of inflow catheter 40 is configured to be disposed in fluid communication with a source of CSF. For example, outlet end 44 may be positioned within CSF of the intrathecal space or in a cerebral ventricle V of brain B of patient P. More specifically, outlet end 44 may be positioned within the arachnoid membrane, the subarachnoid space, or one of the lateral ventricles. The ventricles form a group of interconnected cavities that are located within the cerebral hemispheres and brain stem. These ventricles or spaces are continuous with the central canal of the spinal cord and are similarly filled with CSF that may be absorbed and replenished by the body of the patient. Alternatively, artificial CSF may be infused by system 100 to replenish, flush, or both, CSF in these same spaces.

Outlet end 44 may be configured in any form suitable for placement within brain B so it is capable of depositing artificial CSF in a cerebral ventricle. Conveniently, the form of outlet end 44 may be similar or identical to conventional ventricular catheters of the type used for draining CSF for treating hydrocephalus, such as those described in U.S. Patent Nos. 5,385,541 to Wolff and 4,950,232 to Ruzicka. Additionally, the form of outlet end 44 may be similar or identical to conventional ventricular catheters of the type used for delivering artificial CSF for treating pain or spasticity, such as those described in U.S. Pat. Pub. Nos. 2005/0090549 to Hildebrand and 201110021469 to Meythaler. Suitable ventricular catheters that may be incorporated into systems constructed according to the present invention are available from commercial suppliers, such as Medtronic PS Medical, Goleta, California.

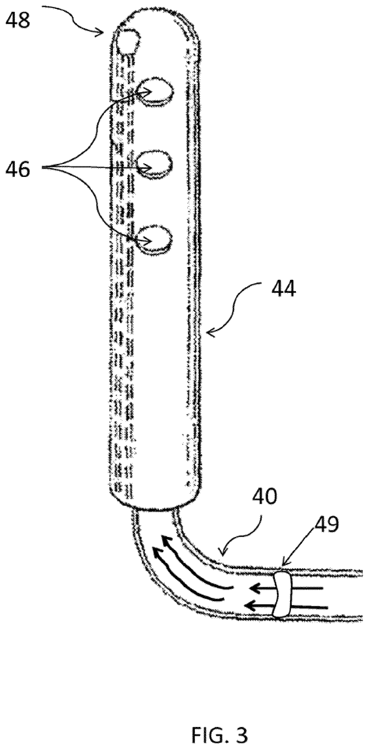

Referring to FIG. 3, one example of outlet end 44 of inflow catheter 40 is described. Outlet end 44 may include multiple perforations or holes 46, which preferably do not extend more than about 1 to 1.5 cm from the tip. Although a particular outlet hole arrangement is shown, other arrangements may be used without departing from the scope of the invention. Outlet end 44 preferably comprises biocompatible material suitable for implantation in the patient such as implant grade low bending modulus material that is generally kink resistant, such as silicone or reinforced silicone, or medical shunt tubing. The tubing may have an outer diameter of about 2.0 mm and an inner diameter of about 0.5-1.5 mm. Outlet end 44 further may comprise a flange configured to promote sealing to the brain, to allow inflow catheter 40 to pass into the cerebral ventricles without fluid leakage.

One or more sensors may be integrated into system 100 for detecting and/or indicating a variety of fluid and/or pump parameters to other components of the system or to the physician or patient. For example, outlet end 44 may further include, or be in communication with, pressure sensor 48, such as a pressure transducer, configured to monitor CSF pressure or ICP, for instance at outlet end 44 of outlet catheter 40, as shown in FIG. 3. Pressure sensor 48 may be disposed in CSF within cerebral ventricle V of brain B and located in the vicinity of the tip of outlet end 44 of outlet catheter 40. Pressure sensor 48 need not be physically connected to the rest of the apparatus but may be in wireless connection therewith. Pressure sensor 48 may be used to monitor the ICP and ensure that the ICP is sufficiently high as to obtain a normal TLCPD. Additionally, the pressure sensor may be used to monitor the ICP and ensure that the ICP is not increased to a level that increases the risk of subdural hematomas or hydrocephalus and midline shifts or that destabilizes the pressure in the ventricles.

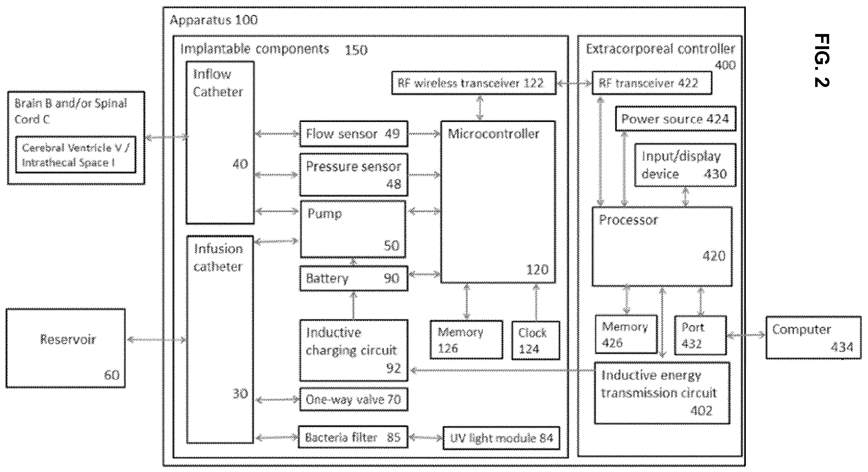

In order to ensure a suitable ICP (and TLCPD), pressure sensor 48 further may be configured to provide an output that is used to control operation of pump 50. For example, pressure sensor 48 may be configured to send a signal to microcontroller 120, in response to sensing a pressure above or below a certain threshold or predetermined amount. Microcontroller 120 may be configured to control the operation of pump 50 (as shown in FIG. 2) by activating or stopping the pump from pumping artificial cerebrospinal fluid from the reservoir to the brain in response to the output of pressure sensor 48. More specifically, microcontroller 120 may activate and stop pump 50 as to obtain an ICP between 11 and 16 mm Hg, preferably up to 15 mm Hg.

In particular embodiments, microcontroller 120 may activate and stop pump 50 as to reduce the TLCPD (i.e. IOP minus ICP), preferably to a value of about 4 mm Hg, preferably even less than 4 mm Hg, such as 2 mm Hg or 1 mm Hg. TLCPD may be determined or estimated based on the ICP as measured by a sensor as described above and an (average) IOP value obtained via a prior measurement, e.g. via tonometry as known in the art.

Inflow catheter 40 may further include flow sensor 49 to detect, measure, and/or monitor the volume and flow rate of artificial CSF pumped into the intrathecal space surrounding the spinal cord and/or into the cerebral ventricles. Flow sensor 49 also may be configured to send a signal to microcontroller 120 regarding the volume and flow rate in order to control pump 50. Flow sensor 49 also may be used to ensure that system 100 is operating properly after implantation and during use.

In a preferred embodiment, microcontroller 120 coordinates and controls operation of the components of system 100. For example, microcontroller 120 may use output signals from pressure sensor 48 and flow sensor 49 to control pump 50 by turning the pump on or off or increasing or decreasing the pump speed (and therefore the fluid flow rate). As a further example, microcontroller 120 may stop pump 50 from pumping artificial CSF from the reservoir into the intrathecal space surrounding the spinal cord and/or into the cerebral ventricles when a specific volume of artificial CSF has been pumped, unless CSF pressure or ICP is less than a threshold pressure.

Microcontroller 120 may be configured to send a signal to power source 424 coupled to pump 50 to indicate when to provide or stop power to pump 50 responsive to output used within system 100 to send signals between the components, such as pressure sensor 48, flow sensor 49, pump 50, and microcontroller 120. Microcontroller 120 further may include memory 126 to record operation of system 100 and/or record a specific algorithm used to infuse the artificial CSF.

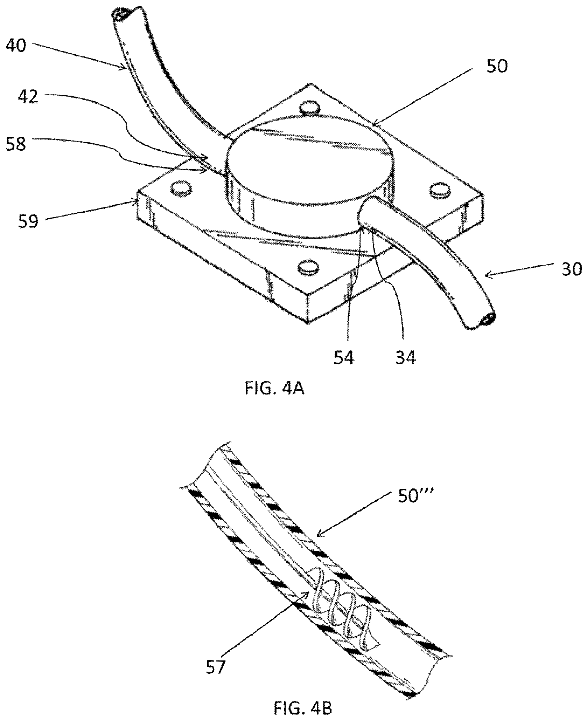

As shown in FIG. 4A, outlet end 34 of infusion catheter 30 is connected to, or coupled with inlet port 54 of pump 50. Outlet port 58 of pump 50 then is connected to inlet end 42 of inflow catheter 40. Pump 50 is configured to control the flow rate and the infusion rate of the fluid (e.g. artificial CSF) being deposited by system 100. More specifically, pump 50 controls the flow rate from reservoir 60 through infusion catheter 30 and into inflow catheter 40.

FIG. 4A shows an embodiment of implantable pump 50 connected to infusion catheter 30 and inflow catheter 40. Pump 50 preferably comprises a battery-powered electromechanical pump. Further, pump 50 may be a positive displacement gear pump as described in U.S. Pat. Pub. No. US 201210209165 to Degen. Alternatively, pump 50 may be a diaphragm pump, piston pump, rotary pump, peristaltic pump, screw pump, or any other suitable pump configuration. Pump 50 also may be remotely operated as is known in the art.

Pump 50 preferably is disposed in a housing manufactured from a suitable biocompatible material, and may include base 59 having suture holes that permit the pump to be fixed to a portion of the patient's anatomy, e.g. within the thorax or peritoneum.

FIG. 4B illustrates an alternative screw pump arrangement, suitable for use in system 100, where screw shaft 57 is mounted for rotation within pump 50'" and the drive is disposed in a hermetically sealed package mounted to the conduit exterior. The drive may be coupled to the screw shaft 57 with a gear transmission as would be apparent to one of ordinary skill in the art. Other screw pump configurations also may be useful, such as those disclosed in U.S. Patent No. 4,857,046 to Stevens et al. or U.S. Patent No. 5,372,573 to Habib.

Pump 50 may be placed and secured anywhere between infusion catheter 30 and inflow catheter 40, although it is preferably implanted at site that provides good accessibility to the surgeon and provides some protection for the device, once implanted. For example, pump 50 may be implanted within the chest or abdomen of the patient. More specifically, pump 50 may be placed in the thoracic cavity and positioned in the lateral mid-thorax near the axillary line and on the under surface of a rib, and may be held in place with sutures to the periosteum.

Referring now to FIG. 2, pump 50 in a preferred embodiment is controlled by microcontroller 120. Pump 50 may operate continuously or periodically to deposit CSF in the intrathecal space surrounding the spinal cord and/or into the cerebral ventricles. For example, pump 50 may operate according to a schedule, time, or program, operate on demand, or operate according to the sensed parameters, such as CSF pressure (ICP) or the volume pumped. Microcontroller 120 may use the output of pressure sensor 48 and/or flow sensor 49 to control the flow rate provided by pump 50, as discussed previously. Alternatively or additionally, pump 50 may maintain an infusion rate of CSF at a rate selected to be equal to the natural daily absorption of CSF by the patient to allow the body to sufficiently absorb CSF and maintain an adequate intracranial pressure. Pump 50 may maintain an infusion rate of the fluid in the range of below 0.1 ml/min, 0.1 ml/min to 0.2 ml/min, 0.2 ml/min to 0.42 ml/min, or 0.42 ml/min to 0.7 ml/min. Alternatively, the pump may be configured to maintain a higher infusion rate, such as 0.7 ml/min (1 L/day) to 1.04 ml/min (1.5 L/day).

Microcontroller 120 may include clock 124 to control pump 50. For example, microcontroller 120 may be programmed to activate the pump periodically in response to clock 124 and to pump a predetermined amount of artificial CSF from reservoir 60 to cerebral ventricle V. The predetermined amount may be based on average or specific CSF infusion rates with respect to particular times of day, or may be specifically titrated for a particular patient.

As depicted in FIGS. IA and 4, infusion catheter 30, which may be similar in design to inflow catheter 40, connects reservoir 60 to pump 50. In particular, outlet end 34 of infusion catheter 30 is coupled with inlet port 54 of pump 50.

Inlet end 32 of infusion catheter 30 is configured to be coupled to reservoir 60, so that artificial CSF is drawn through inlet end 32 into pump 50. As described above, reservoir 60 may be external to the patient's body or implanted under the skin of the patient with means for receiving additional artificial CSF.

One-way valve 70 may be positioned along infusion catheter 30 or inflow catheter 40 to provide unidirectional flow of artificial CSF within system 100. More specifically, one-way valve 70 allows the fluid to flow in only one direction: from the reservoir to the brain or spine. This prevents any backflow to the reservoir of harmful proteins from the brain. One-way valve 70 may be located within or on infusion catheter 30 or inflow catheter 40 or more preferably, may be housed within pump 50.

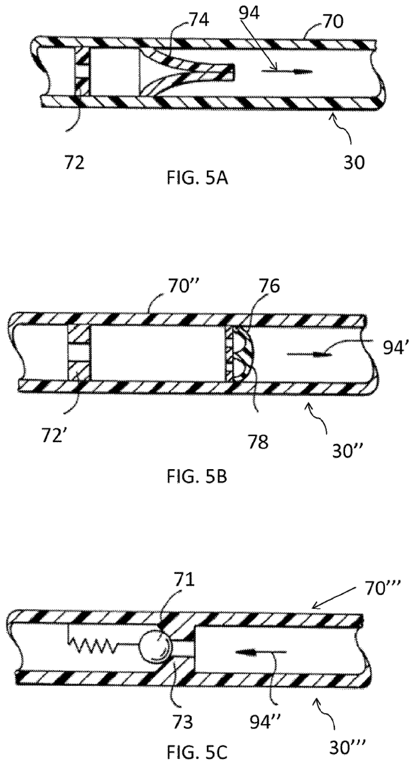

Examples of one-way valves suitable for use in system 100 are shown in FIGS. 5A-5C. In each of the examples, fluid may flow freely in the direction of arrow 94. However, fluid flow opposite to the direction of arrow 94 will force one-way valve 70 to close, thereby preventing backflow. In order to re-open one-way valve 70, sufficient pressure in the direction of arrow 94 must be provided, thus ensuring that fluid moves only in the correct direction.

As shown in FIG. 5A, one-way valve 70 may comprise orifice plate 72 in series with one-way valve 70, illustratively, duck-bill valve 74. Both orifice plate 72 and duck-bill valve 74 may be mounted within infusion catheter 30, inflow catheter 40 or the housing of pump 50. Flow in the direction of arrow 94 will open duck-bill valve 74 and permit fluid flow through infusion catheter 30 and inflow catheter 40. One-way valve 70 alternatively or additionally may comprise a variety of other flow restrictive elements, such as a multiple orifice plate, a filter element, or any other discrete element or combination of elements that may provide a flow resistance capable of yielding the flow rates described herein.

FIG. 5B depicts another embodiment of one-way valve 70" comprising orifice plate 72' in series with umbrella valve 76. Umbrella valve 76 includes an elastomeric membrane 78 that opens under pressure to permit flow in the direction of arrow 94'.

FIG. 5C depicts yet another embodiment of one-way valve 70'" comprising spring-loaded ball valve 71 disposed in valve seat 73. Valve seat 73 also serves as an orifice to limit flow through the assembly and control the direction of the fluid flow. Flow in direction of arrow 94" will open ball valve 71 and permit flow through the orifice defined by valve seat 73.

In the above cases, the orifice may be selected to provide a desired flow rate when the patient is in a vertical position. One-way valve 70 will be implanted within the patient with a known orientation, usually vertical, in order to provide a known pressure head of artificial CSF onto orifice 72 or 73. This pressure will be sufficient to open the associated one-way valve 70 and flow will be established when the patient is in an upright position. Suitable orifice diameters in the range from 0.03 mm to 0.4 mm, preferably from 0.1 mm to 0.2 mm, for orifices having a thickness in the range from 0.001 mm to 100 mm, preferably from 1 mm to 5 mm, in order to establish average hourly flow rates in the range from 0.5 ml/hour to 15 ml/hour, preferably 1 ml/hour to 3 ml/hour.

A bacterial filter 85 may be included between inlet end 32 of infusion catheter 30 and outlet end 44 of inflow catheter 40 to prevent bacteria from migrating through system 100 to the patient's brain as depicted in FIG. IA. Although one-way valve 70 is located along infusion catheter 30 or inflow catheter 40, bacterial filter 85 may be desirable to further prevent bacteria from reaching the brain in the event of malfunction of pump 50. Bacterial filter 85 may be incorporated in the housing of pump 50, and may include ultraviolet ("UV") light module 84 configured to irradiate artificial CSF and destroy bacteria passing within infusion catheter 30 and inflow catheter 40. Optionally, bacterial filter 85 may be replaced by antibiotic or antimicrobial coatings disposed on or impregnated within some or all of the components of system 100.

Referring again to FIG. 2, system 100 may include extracorporeal controller 400 that communicates wirelessly with implantable components 150. Extracorporeal controller 400 may provide power to implantable components 150 and/or control activation of the implantable components, such as pump 50.

Implantable components 150 may be powered by battery, or alternatively by a super-capacitor, or other energy storage device. In a preferred embodiment, the power/energy source may be rechargeable. For example, battery 90 may be coupled to implantable inductive charging circuit 92 configured to receive energy from inductive energy transmission circuit 402 of extracorporeal controller 400.

Microcontroller 120 may be coupled to a first transceiver, such as radio frequency (RF) wireless transceiver 122. Extracorporeal controller 400 may be coupled to a second transceiver, such as RF transceiver 422. RF wireless transceiver 122 and RF transceiver 422 may bi-directionally communicate information, such as the operation of the pump, CSF pressure or ICP, and/or the desired infusion rate of the artificial CSF. For example, microcontroller 120 may receive programmed instructions from extracorporeal controller 400 relating to pump activation intervals, targeted volumes of CSF to be pumped and desired flow rates. Additionally, extracorporeal controller 400 may receive data or information from microcontroller 120 relating to pump activation periods, measured pressures, and actual volumes of artificial CSF pumped through inflow catheter 40.

Extracorporeal controller 400 preferably includes processor 420 to coordinate and control its various components and functions. Extracorporeal controller 400 further may include power source 424 to power the extracorporeal controller (and potentially also implantable components 150), and may comprise a battery or an electrical outlet. Extracorporeal controller 400 further may include memory 426 to record information, such as the information received from implantable components 150 or a specific algorithm to convey to the implantable components regarding the infusion of artificial CSF to the brain.

In order for the patient or the physician to enter information into system 100 or for system 100 to display information, extracorporeal controller 400 preferably includes input/display device 430 and/or port 432 to connect to computer 434, such as a laptop computer. Input/display device 430 may include indicators or a control interface to control system 100 and display detailed information about the system. Extracorporeal controller 400 optionally may wirelessly conveyor receive information from computer 434, such as whether system 100 is properly functioning, the current (and past) CSF pressures, the volume of artificial CSF injected, the current (and past) flow rate of artificial CSF through the system, and/or whether pump 50 is currently activated. This information may be conveyed to the patient or physician as a visual message or indicator signal, such as a light or audible signal, that is initiated once pump 50 has been activated. Computer 434 may optionally provide power to extracorporeal controller 400.

## Claims

1. A composition comprising cerebrospinal fluid (CSF) or a CSF-like composition for use in the prevention and/or treatment of glaucoma, wherein the CSF-like composition is artificial CSF (aCSF), wherein aCSF comprises sodium ions at a concentration of 140-190 mM, potassium ions at a concentration of 2.5-4.5 mM, calcium ions at a concentration of 1-1.5 mM, magnesium ions at a concentration of 0.5-1.5 mM, phosphor ions at a concentration of 0.5-1.5 mM, chloride ions a concentration of 100-200 mM.

2. The composition for use according to claim 1, wherein said prevention or treatment of glaucoma is ensured by infusion into the intrathecal space or the cerebral ventricles.

3. The composition for use according to claim 2, wherein said infusion ensures reduction of the trans-lamina cribrosa pressure difference (TLCPD).

4. The composition for use according to claim 3, wherein said infusion reduces the TLCPD to about 4 mmHg or less, preferably to 1 mmHg or 2 mmHg.

5. The composition for use according to claim 3 or 4, wherein said reduction of TLCPD is ensured by an increase of intracranial pressure (ICP).

6. The composition for use according to claims 2 to 5, wherein said infusion ensures an intracranial pressure of between 11 and 16 mmHg.

7. The composition for use according to any one of claims 2 to 6, wherein said infusion ensures an increase in CSF turnover.

8. The composition for use according to any one of claims 2 to 7, wherein said infusion is ensured by an implantable pump.

## Patentansprüche

1. Zusammensetzung, die eine Cerebrospinalflüssigkeit (CSF) oder eine CSF-ähnliche Zusammensetzung umfasst, zur Verwendung bei der Vermeidung und/oder Behandlung eines Glaukoms, wobei die CSF-ähnliche Zusammensetzung eine künstliche CSF (aCSF) ist, wobei die aCSF Natriumionen in einer Konzentration von 140-190 mM, Kaliumionen in einer Konzentration von 2,5-4,5 mM, Calcium-Ionen in einer Konzentration von 1-1,5 mM, Magnesiumionen in einer Konzentration von 0,5-1,5 mM, Phosphorionen in einer Konzentration von 0,5-1,5 mM, Chlorid-Ionen in einer Konzentration von 100-200 mM umfasst.

2. Zusammensetzung zur Verwendung nach Anspruch 1, wobei die Vermeidung oder Behandlung des Glaukoms durch Infusion in den Intrathekalraum oder in die Gehirnventrikel sichergestellt wird.

3. Zusammensetzung zur Verwendung nach Anspruch 2, wobei die Infusion eine Verringerung des Trans-Lamina cribrosa-Druckunterschieds (trans-lamina cribrosa pressure difference; TLCPD) sicherstellt.

4. Zusammensetzung zur Verwendung nach Anspruch 3, wobei die Infusion den TLCPD auf etwa 4 mmHg oder weniger verringert, vorzugsweise auf 1 mmHg oder 2 mmHg.

5. Zusammensetzung zur Verwendung nach Anspruch 3 oder 4, wobei die Verringerung des TLCPD durch eine Erhöhung des intrakraniellen Drucks (intracranial pressure; ICP) sichergestellt wird.

6. Zusammensetzung zur Verwendung nach den Ansprüchen 2 bis 5, wobei die Infusion einen intrakraniellen Druck zwischen 11 und 16 mmHg sicherstellt.

7. Zusammensetzung zur Verwendung nach einem der Ansprüche 2 bis 6, wobei die Infusion eine Erhöhung des CSF-Umsatzes sicherstellt.

8. Zusammensetzung zur Verwendung nach einem der Ansprüche 2 bis 7, wobei die Infusion durch eine implantierbare Pumpe sichergestellt wird.

## Revendications

1. Composition comprenant du liquide céphalorachidien (LCR) ou une composition analogue à du LCR à utiliser dans la prévention et/ou le traitement du glaucome, dans laquelle la composition analogue à du LCR est du LCR artificiel (aLCR), dans laquelle l'aLCR comprend des ions sodium à une concentration de 140 à 190 mM, des ions potassium à une concentration de 2,5 à 4,5 mM, des ions calcium à une concentration de 1 à 1,5 mM, des ions magnésium à une concentration de 0,5 à 1,5 mM, des ions phosphore à une concentration de 0,5 à 1,5 mM, des ions chlorure à une concentration de 100 à 200 mM.

2. Composition à utiliser selon la revendication 1, dans laquelle ladite prévention ou ledit traitement du glaucome est assuré(e) par perfusion dans l'espace intrathécal ou les ventricules cérébraux.

3. Composition à utiliser selon la revendication 2, dans laquelle ladite perfusion assure la réduction de la différence de pression trans-lamina cribrosa (TLCPD).

4. Composition à utiliser selon la revendication 3, dans laquelle ladite perfusion réduit la TLCPD à environ 4 mmHg ou moins, de préférence à 1 mmHg ou 2 mmHg.

5. Composition à utiliser selon la revendication 3 ou 4, dans laquelle ladite réduction de TLCPD est assurée par une augmentation de pression intracrânienne (ICP).

6. Composition à utiliser selon les revendications 2 à 5, dans laquelle ladite perfusion assure une pression intracrânienne comprise entre 11 et 16 mmHg.

7. Composition à utiliser selon l'une quelconque des revendications 2 à 6, dans laquelle ladite perfusion assure une augmentation du renouvellement de LCR.

8. Composition à utiliser selon l'une quelconque des revendications 2 à 7, dans laquelle ladite infusion est assurée par une pompe implantable.
